# EUROPÄISCHE PATENTSCHRIFT

(11) **EP 0 777 482 B1**
(45) Veröffentlichungstag und Bekanntmachung des Hinweises auf die Patenterteilung: **14.11.2001**
(21) Anmeldenummer: 95930441.1
(22) Anmeldetag: 07.08.1995
(51) Int. Cl.: A61K 31/55, A61K 31/52, A61K 38/13, A61K 45/06

(54) **KOMBINATIONSPRÄPARAT ZUR ANWENDUNG BEI IMMUNOLOGISCHEN ERKRANKUNGEN**
COMBINED PREPARATION FOR THE THERAPY OF IMMUNE DISEASES
PREPARATION COMBINEE POUR LE TRAITEMENT DE MALADIES IMMUNITAIRES

(30) Priorität: 25.08.1994 DE 4430128
(43) Veröffentlichungstag der Anmeldung: 11.06.1997
(73) Patentinhaber: HOECHST AKTIENGESELLSCHAFT, 65929 Frankfurt am Main (DE)
(72) Erfinder: SCHÖNHARTING, Martin, D-65232 Taunusstein (DE); MÜLLNER, Stefan, D-65239 Hochheim (DE); ZABEL, Peter, D-23795 Segeberg (DE)
(86) Internationale Anmeldenummer: EP9503125
(87) Internationale Veröffentlichungsnummer: WO9605838

(56) Entgegenhaltungen:
- EP-A- 0 234 262
- EP-A- 0 490 181
- EP-A- 0 493 682
- EP-A- 0 544 391
- ANNUAL MEETING OF THE CANADIAN SOCIETY FOR CLINICAL INVESTIGATION AND THE ROYAL COLLEGE OF PHYSICIANS AND SURGEONS OF CANADA, OTTAWA, ONTARIO, CANADA, SEPTEMBER 11-14, 1992. CLIN INVEST MED, 15 (4 SUPPL.). 1992. A61., REECE D E ET AL 'CHRONIC GRAFT VERSUS-HOST DISEASE CGVHD IN PATIENTS PTS RECEIVING UNRELATED DONOR UD ALLOGENEIC BONE MARROW TRANSPLANTS ALLO BMTS INCIDENCE RISK FACTORS AND OUTCOME'
- AM J CLIN ONCOL, Bd. 17, Nr. 4, August 1994 Seiten 313-316, D.J. STEWART ET AL. 'Addition of Pentoxifylline plus Nifedipine to chemotherapy in patients with Cisplatin-resistant cancers of the lung and other sites.'
- J CARDIOVASC PHARMACOL, Bd. 18, Nr. 5, 1991 Seiten 761--768, G. BERKENBOOM ET AL. 'Prevention of Cyclosporine A-induced vascular toxicity by Pentoxifylline'
- FUNDAM CLIN PHARMACOL, Bd. 8, Nr. 1, 16.Februar 1994 Seiten 26-33, B. SARRIA ET AL. 'The Nicardipine-Isoprenaline interaction in human and guinea-pig isolated airways'
- BIOCHEM PHARMACOL, Bd. 33, Nr. 17, 1984 Seiten 2723-2726, K.S. GALDAL ET AL. 'Inhibition of the thromboplastin response of endothelial cells in vitro'
- TRANSPLANT PROC, OCT 1994, 26 (5) P2745-6, UNITED STATES, CARRIER M ET AL 'Effect of pentoxifylline on renal toxicity of cyclosporine: preliminary results.'
- THE 9TH INTERNATIONAL CONGRESS OF IMMUNOLOGY;MEETING SPONSORED BY THE AMERICAN ASSOCIATION OF IMMUNOLOGISTS AND THE INTERNATIONAL UNION OF IMMUNOLOGICAL SOCIETIES, SAN FRANCISCO, CALIFORNIA, USA, JULY 23-29, 1995; CONFERENCE PAPER, Seite 852 KHABAR K S A ET AL 'Pentoxifylline selectively potentiates cyclosporin A (CYA)-mediated suppression of mixed lymphocyte cytotoxicity (MLC) among other immunological functions'

## Beschreibung

Die Erfindung betrifft pharmazeutische Kombinationspräparate zur Behandlung von immunologischen Erkrankungen im weitesten Sinne, die ursächlich oder symptomatisch verschiedene Krankheitsbilder bestimmen. Diese Kombinationspräparate sind durch die den Einzelkomponenten jeweils zugrundeliegenden pharmakologischen Wirkmechanismen festgelegt und lassen sich durch eine
- Hemmwirkung auf Phosphodiesterasen
kombiniert mit einer
- Erniedrigung der biologisch effektiven intrazellulären Ca²⁺-Konzentration
charakterisieren.

Die Aktivierung von immunkompetenten Zellen, welches die Grundlage jeglicher Immunantwort ist, läuft über die sogenannte Signalkette ab: Ein extrazellulärer Stimulus (z.B. Toxin, Antigen, aber auch Complement, Entzündungsmediatoren, Arachidonsäure-Derivate oder andere Stoffwechselprodukte) erreicht als "Informationsträger" die Zelle und gibt die stoffliche Information - meist über geeignete Rezeptoren - an die Zielzelle ab. Intrazellulär wird diese Information über verschiedene Zwischenstufen im Rahmen der Signalkette bis in den Kern weitergegeben, der auf diesen Reiz mit Proliferation und/oder Bildung spezifischer Aktivatoren reagiert, womit die Immunantwort auf geeignete Weise in Gang kommt. Die einzelnen Schritte in dieser intrazellulären Weitergabe der Information sind heute allerdings noch wenig geklärt, zumal es offenbar verschiedene Wege gibt, die innerhalb der Zelle beschritten werden können, um eine Immunantwort auszulösen. So lassen sich beispielsweise in Abhängigkeit von Art des Rezeptors und der daran angekoppelten Untereinheiten (Guaninnukleotid-bindende Proteine, sogenannte "G-Proteine") in der Folge Inositoltriphosphat (IP₃), Diacylglycerol (DAG), Phosphatidylcholin (PC) oder auch Phosphatidylinositol (PI) nachweisen, die durch eine Reiz-induzierte Aktivierung von Phospholipase C oder D entstanden sind und mit einer Erhöhung von intrazellulärem freiem Ca²⁺ einhergehen, während eine Aktivierung von Phospholipase A₂ die Bildung von Arachidonsäure-Derivaten (Prostaglandine, Leukotriene) nach sich zieht, die über geeignete Rezeptoren ihrerseits einen eigenen Reiz mit entsprechenden Folgeerscheinungen auslösen können. Andere Rezeptoren wieder sind über entsprechende G-Proteine an Adenylat-Cyclase (AC) gekoppelt, deren Aktivierung in der Bildung von zyklischem
3',5'-Adenosinmonophosphat (cAMP) resultiert, während dritte wieder hiervon unabhängige Effekte auszulösen scheinen (Übersicht bei Roitt (ed.): Essential Immunology. Blackwell Scient. Publ. Oxford 1991 sowie Foreman, Fan (eds.): Textbook of Immunopharmacology. Blackwell Scient. Publ. Oxford 1994).

Sowohl Ca²⁺ als auch cAMP stellen somit wichtige Informationsübermittler ("second messenger") im Rahmen der Signaltransduktion dar, wobei die im weiteren Verlauf intrazellulär sich abspielenden Vorgänge jedoch so komplex sind, daß eine generelle Vorhersage der resultierenden zellbiologischen Ereignisse unmöglich ist. Immerhin scheinen die meisten nachgeschalteten Aktivierungsphänomene von Phosphorylierungsschritten in der Zelle abzuhängen, die ihrerseits wieder über entsprechende Proteinkinasen oder Phosphatasen reguliert werden. Hier ist insbesondere die Proteinkinase C (PKC) zu nennen, deren Aktivität über DAG und IP₃/Ca²⁺ in einer konzertierten Aktion gesteuert wird, wodurch der Verfügbarkeit von intrazellulärem freiem Ca²⁺ eine Schlüsselrolle in der Kaskade der Zellaktivierung zukommt, ungeachtet der Tatsache, daß im weiteren Verlauf meist der Ca²⁺/Calmodulin-Komplex die Ca²⁺-Effekte vermittelt (Hidaka et al.: Cell Calcium 13, 465-472 (1992); Kun et al.: Endocrin. Rev. 14, 40-58 (1993)). Phosphorylierungen müssen aber nicht in jedem Falle zu Aktivierungen führen, sondern können auch Hemmeffekte nach sich ziehen. So wirkt die cAMP-abhängige Proteinkinase A (PKA) beispielsweise der Zellaktivierung entgegen, wie überhaupt erst das komplexe Wechselspiel von Phosphorylierung und Dephosphorylierung eine effiziente Regulation der Zellaktivität erlaubt und es auf diese Weise dem Organismus ermöglicht, sinnvoll auf wechselnde Einflüsse von außen zu reagieren (Sitkovsky et al.: Ann. NY Acad. Sci. 532, 350-358 (1988); Takayama et al.: J. Pharm. Sci. 78, 8-10 (1989)). Dabei kann ein und dieselbe Substanz sowohl agonistische wie auch antagonistische Effekte aufweisen. So hemmt cAMP beispielsweise die Interleukin-2 (IL-2)-induzierte Aktivierung (Friedrich et al.: Eur. J. Immunol. 19, 1111-1116 (1979)), potenziert dagegen aber nicht nur die IL-1-induzierte Aktivierung, sondern kann unter Umständen sogar selber als IL-1-Agonist wirken (Shirakawa et al.: Proc. Natl. Acad. Sci. 85, 8201-8205 (1988); Schlegel-Häuter et al.: Cell. Sign. 2, 489-496 (1990)). Bei derartigen mechanistischen Betrachtungen ist jedoch zu berücksichtigen, daß das Auftreten eines "second messengers" auf einen entsprechenden Reiz hin alleine noch keine Rückschlüsse auf dessen funktionelle Beteiligung erlaubt; vielmehr kann dies auch ein bloßes Epiphänomen darstellen (Hansen et al.: Brit. J. Cancer 69, 291-298 (1994); Baumgold et al.: J. Neurochem. 58, 1754-1759 (1992)). Diese Aussage wird exemplarisch durch Beispiel 3 dieser Patentanmeldung gestützt, wo gezeigt wird, daß der Effekt eines Phosphodiesterase-Hemmers (Pentoxifyllin) durchaus nicht immer mit einer Erhöhung von cAMP erklärt werden kann, sondern unter bestimmten Bedingungen vielmehr in der generellen Hemmwirkung dieser Substanzgruppe auf Dephosphorylierungsvorgänge zu suchen ist. Es hängt demnach von vielfältigen Faktoren ab, inwieweit bestimmte Mechanismen im Rahmen der Signaltransduktion Bedeutung erlangen können; entsprechend unvorhersehbar sind die Effekte von Substanzen, die an verschiedenen Stellen am Geschehen beteiligte Signalsubstanzen pharmakologisch beeinflussen, insbesondere wenn sie in Kombination einwirken.

Substanzen mit Phosphodiesterase-Hemmwirkung sind bekannt und werden teilweise schon therapeutisch eingesetzt. Sie stellen eine heterogene Substanzklasse dar, die dadurch gekennzeichnet ist, daß sie die Spaltung von zyklischen Mononucleotiden durch Phosphodiesterasen (PDE) hemmen, wodurch sich cAMP oder cGMP intrazellulär anreichert; es muß jedoch betont werden, daß sich die Hemmwirkungen von PDE-Hemmern (wie oben bereits angesprochen) aber nicht hierin erschöpft, sondern sich generell auf unterschiedlichste Enzyme mit wie auch immer gearteter Phosphatase-Aktivität erstreckt, mit allen sich daraus ableitenden Konsequenzen auf die vielfältigen regulatorischen (De-) Phosphorylierungs-Vorgänge innerhalb der Zelle. Aber selbst was die engere Reaktion der Spaltung einer Phosphodiester-Bindung anbelangt, so wird diese Reaktion nicht von einem einzigen Enzym, sondern von einem ganzen Enzymsystem mit mindestens 5 verschiedenen Isoenzymfamilien und mehr als 20 individuellen Enzymen katalysiert (Übersicht bei Beavo et al.: Trends Pharmacol. Sci. 11, 150-155 (1990)). Da die zugrundliegende enzymatische Reaktion (= hydrolytische Spaltung einer Phosphoester-Bindung) aber stets dieselbe ist, weisen alle PDE-Hemmer eine entsprechend überlappende Hemmwirkung auf; so gibt es z.B. PDE-Inhibitoren wie beispielsweise Theophyllin, Pentoxifyllin oder auch Papaverin, die recht unspezifisch auch sehr unterschiedliche Phosphodiesterasen hemmen. Aber selbst wenn PDE-Hemmer in der Fachliteratur als "spezifisch" bezeichnet werden, so ist hier lediglich eine gewisse Präferenz für eine bestimmte Isoenzym-Familie gemeint, ohne daß sich hieraus gleich ein Ausschließlichkeits-Anspruch ableiten ließe. Beispiele hierfür gibt es viele: So spaltet die Ca²⁺/Calmodulin-abhängige PDE I sowohl cGMP als auch cAMP und wird z.B. durch Phenothiazin, Vinpocetin oder IBMX gehemmt. Die cGMP-stimulierbare PDE II spaltet ebenfalls cGMP und cAMP, allerdings sind für dieses Enzym keine selektiven Inhibitoren bekannt im Gegensatz zur PDE III, die eine gleichartige Substratspezifität wie PDE II aufweist, aber durch cGMP und eine Vielzahl weiterer Substanzen gehemmt werden kann. Die zum Teil erheblichen strukturellen Unterschiede zwischen PDE III-Hemmern wie Cilostamid, Milrinon, Trequinsin, Indolidan oder Quazinon lassen vermuten, daß es einerseits verschiedene Hemmbereiche auf der PDE III, andererseits aber auch verschiedenartige Isoenzyme dieser PDE-Familie geben düfte. Weiterhin finden sich als Hemmer der PDE IV, die eine starke Präferenz für die Hydrolyse von cAMP aufweist, in der Literatur z.B. Ro 201724 und Rolipram, während die für die Spaltung von cGMP spezifische PDE V durch Zaprinast oder auch Dipyridamol gehemmt wird.

Auch Substanzen, die den Gehalt an biologisch effektivem intrazellulärem Ca²⁺ erniedrigen können, sind bekannt und werden vielfach schon therapeutisch genutzt. Hierunter fallen einmal die klassischen Kalziumkanalblocker, die - basierend auf den Kenntnissen über die verschiedenen Arten der Ca²⁺-Kanäle - in Dihydropyridine (z.B. Nifedipin, Nicardipin, Nimodipin), Phenylalkylamine (z.B. Verapamil) und Benzothiazepine (z.B. Diltiazem) eingeteilt werden können (Übersicht bei Piepho: Hosp. Pharm. 26, 856-864 (1991); Luft et al.: Clin. Exp. Hypertens. 15, 1263-1276 (1993)) und deren gezielte Weiterentwicklungen wie Isradipin, Felodipin, Diperdipin oder Amlodipin. Aber auch atypische Ca²⁺-Kanal-Blocker wie Flunarizin, Fluspirilen, Pimozid, Fantofaron, Nicergolin, Cyclandelat oder Antibiotika auf Aminoglycosid-Basis wie Neomycin, Gentamycin oder Kanamycin kamen im Einzelfall schon bis zur klinischen Einsatzreife. Andere Ansätze wiederum hatten die Hemmung der Freisetzung von Ca²⁺ aus seinen intrazellulären Speichern zum Ziel (Massingham et al.: Drugs Today 27, 459-477 (1991)). Substanzen, die diesen Anforderungen nachkommen, sind beispielsweise Ryanodin, Dantrolen oder TMB-8, haben aber momentan lediglich experimentelle Bedeutung zur Untersuchung des Wirkungsmechanismus genauso wie HA1077, das neben der Hemmung der intrazellulären Ca²⁺-Mobilisation aber offenbar auch direkte Ca²⁺-antagonistische Effekte aufweist. Dies leitet über zur Gruppe der eigentlichen Kalzium-Antagonisten, die entweder freie Ca²⁺-Ionen direkt über eine spezifische Chelatbildung abfangen wie beispielsweise EGTA und dessen Abkömmlinge (Übersicht bei Grynkiewicz et al.: J. Biol. Chem. 260, 3340-3346 (1985)) oder aber als Calmodulin-Antagonisten wirken. Zu letzteren gehören die Derivate der Naphthalinsulfonsäure W7, W12 und W13, Calmidazolium oder Fluphenazin genauso wie die Gruppe der Cyclosporine oder Tacrolimus, die allerdings erst an intrazelluläre "Immunophiline" gebunden sein müssen, bevor sie das Ca²⁺/Calmodulin-abhängige Enzym Calcineurin (eine Phosphatase) hemmen können (Liu: Immunol. Today 14, 290-295 (1993)). Auch Kombinationen von sogenannten "PDE-Hemmern" mit sogenannten "Kalzium-Antagonisten" im weitesten Sinne sind bereits bekannt. Dies ergibt sich aus dem Indikationsgebiet beider Substanzgruppen, das in beiden Fällen vorwiegend den kardiovaskulären Bereich betrifft, so daß die Idee einer Comedikation naheliegt (EP-B-0 234 262). Dieses betrifft jedoch ausschließlich die Behandlung von Durchblutungsstörungen aufgrund im Vergleich zu den Einzelkomponenten gesteigerter spasmolytischer und mikrozirkulatorischer Effekte der Kombination und weist keinerlei Bezug auf immunologische Fragestellungen auf, wie generell der Einsatz der Einzelkomponenten auf dem immunologischen Gebiet - von wenigen Ausnahmen wie Cyclosporin oder Tacrolimus abgesehen - sich aufgrund von Unverträglichkeitserscheinungen bei den dann notwendigen höheren Dosierungen meist verbietet (Fisher et al.: Drugs 46, 961-975 (1993)). Ferner wird die Verwendung von PDE-Hemmern (bestimmte Xanthinderivate) zusammen mit sogenannten "Kalzium-Antagonisten" (Cyclosporin A oder Tacrolimus) beschrieben (EP 0 493 682, EP 0 490 181, WO 93/17684), die Ansprüche bestehen hier jedoch ausschließlich in der Reduzierung der Toxizität der besagten Immunsuppressiva. Derartige Befunde sind literaturbekannt und beinhalten insbesondere keinerlei Hinweis auf eine irgendwie geartete Verstärkung des immunsuppressiven Effekts dieser speziellen Calmodulin-Antagonisten durch den verwendeten PDE-Hemmer (siehe z.B. Brunner et al.: Renal Fail. 11, 97-104 (1989); Berkenboom et al.: J. Cardiovasc. Pharmacol. 18, 761-768 (1991); Rossi et al.: Drug Saf. 9, 104-131 (1993)).

Es wurde nun gefunden, daß eine Kombination enthaltend eine Verbindung, die eine Hemmungwirkung auf Phosphodiesterasen ausübt, und eine Verbindung, die den biologisch effektiven intrazellulären Ca²⁺-Gehalt vermindert, einen überadditiven immunsuppressiven Effekt aufweist. Aufgrund des Ausmaßes dieses Effekts läßt sich die Anwendung dieser Kombination auf Bereiche ausdehnen, die einer immunsuppressiven Therapie durch die Einzelkomponenten aufgrund von nicht tolerablen Unverträglichkeitserscheinungen bislang verschlossen waren. Gleichzeitig ist davon auszugehen, daß die Therapiekosten über eine Dosiserniedrigung bei gleichbleibender Wirksamkeit in nennenswertem Umfang gesenkt werden können.

Die Erfindung betrifft daher ein Kombinationspräparat, enthaltend mindestens
1) eine Verbindung, die eine Phosphodiesterase-Hemmwirkung aufweist (sogenannte "PDE-Hemmer"),
2) eine Verbindung, die den biologisch effektiven intrazellulären Ca²⁺-Gehalt vermindert (sogenannte Kalzium-Antagonisten), und
3) einen pharmazeutischen Träger,
mit überadditiver Steigerung der immunsuppressiven Wirkung zur gleichzeitigen, getrennten oder zeitlich abgestuften Anwendung.

Bekannte Verbindungen mit Phosphodiesterase-Hemmwirkung sind beispielsweise:
Phenothiazin, Vinpocetin, Cilostamid, Milrinon, Trequinsin , Indolidan, Quazinon, Ro 201724, Rolipram, Zaprinast, Dipyridamol, Papaverin oder Xanthinderivate wie Pentoxifyllin, Theophyllin oder IBMX.

Verbindungen, die den intrazellulären Gehalt an biologisch effektiven Ca²⁺ vermindern, sind beispielsweise:
1. Spezifische Ca²⁺-Kanal-Blocker (Typ P-, L-, N-), z.B. Dihydropyridine wie Nifedipin, Nicardipin, Nimodipin, NZ-105, S11568, Amlodipin, Felodipin, Isradipin oder Diperdipin, Phenylalkylamine wie Verapamil, Benzothiazepine wie Diltiazem, atypische Ca²⁺-Kanal-Blocker wie Flunarizin, Fluspirilen, Pimozid, Fantofaron, Nicergolin, Cyclandelat oder auch Antibiotika auf Aminoglycosid-Basis wie Neomycin, Gentamycin oder Kanamycin;
2. Eigentliche Ca²⁺-Antagonisten, z.B 1,2-Bis-(2-aminoethoxyethan)-N,N,N',N'-tetraessigsäure (EGTA) oder HA1077;
3. Inhibitoren der Ca²⁺-Freisetzung aus intrazellulären Speichern, z.B. Ryanodin, Dantrolen, TMB-8 oder HA1077;
4. Calmodulin-Inhibitoren, z.B. Naphthalinsulfonsäurederivate, Calmidazolium, Fluphenazin, Cyclosporine oder Tacrolimus.

Unter dem Begriff "überadditiv" werden Wirkungen verstanden, die größer als die Summe der Einzelwirkungen sind.

Bevorzugte Kombinationspräparate enthalten Pentoxifyllin und Nifedipin, Pentoxifyllin und Diltiazem, Pentoxifyllin und Verapamil oder Pentoxifyllin und Cyclosporin A.

Das erfindungsgemäße Kombinationspräparat eignet sich beispielsweise zur Behandlung von
- akuten immunologischen Ereignissen wie Sepsis, Allergie, Graft-versus-Host- und Host-versus-Graft-Reaktionen
- Autoimmunerkrankungen, insbesondere rheumatoider Arthritis, systemischem Lupus erythematodes, multipler Sklerose
- Psoriasis, atopischer Dermatitis, Asthma, Urticaria, Rhinitis, Uveitis
- Typ II-Diabetes
- Leberfibrose, zystischer Fibrose, Colitis

Das erfindungsgemäße Kombinationspräparat kann auch Kombinationspackungen oder Kompositionen umfassen, in denen die Bestandteile nebeneinandergestellt sind und deshalb gleichzeitig, getrennt oder zeitlich abgestuft an ein und demselben menschlichen oder tierischen Körper angewendet werden können.

Die Erfindung betrifft ferner die Verwendung einer Kombination einer Verbindung, die eine Phosphodiesterase-Hernmwirkung aufweist, und einer Verbindung, die den biologisch effektiven intrazellulären Ca²⁺-Gehalt vermindert, zur Herstellung eines Arzneimittels mit überadditiver Steigerung der immunsuppressiven Wirkung.

Die Erfindung betrifft ferner ein Verfahren zur Herstellung des Kombinationspräparats, das dadurch gekennzeichnet ist, daß man
1) eine Verbindung, die eine Phosphodiesterase-Hemmwirkung aufweist,
2) eine Verbindung, die den biologisch effektiven intrazellulären Ca²⁺-Gehalt vermindert, und
3) einen pharmazeutischen Träger
in üblicher Weise zu einer pharmazeutischen Darreichungsform verarbeitet.

Das erfindungsgemäße Kombinationspräparat kann als Dosiereinheit in Form von Arzneiformen wie Kapseln (einschließlich Mikrokapseln, die im allgemeinen keine pharmazeutischen Träger enthalten), Tabletten (einschließlich Dragees und Pillen) oder Zäpfchen vorliegen, wobei bei Verwendung von Kapseln das Kapselmaterial die Funktion des Trägers wahrnehmen und der Inhalt z.B. als Pulver, Gel, Emulsion, Dispersion oder Lösung vorliegen kann. Besonders vorteilhaft und einfach ist es jedoch, orale (perorale) Formulierungen mit den beiden Wirkstoffkomponenten 1) und 2) herzustellen, die die berechneten Mengen der Wirkstoffe zusammen mit jedem gewünschten pharmazeutischen Träger enthalten. Auch eine entsprechende Formulierung (Zäpfchen) für die rektale Therapie kann angewandt werden. Ebenso ist die transdermale Applikation in Form von Salben oder Cremes, parenterale (intraperitoneale, subkutane, intravenöse, intraarterielle, intramuskuläre) Injektion oder Infusion von Lösungen oder orale Applikation von Lösungen, die die erfindungsgemäßen Kombinationen enthalten, möglich.
Salben, Pasten, Cremes und Puder können neben den Wirkstoffen die üblichen Trägerstoffe enthalten, z.B. tierische und pflanzliche Fette, Wachse, Paraffine, Stärke, Traganth, Zellulosederivate, Polyethylenglykole, Silicone, Bentonite, Talkum, Zinkoxid, Milchzucker, Kieselsäure, Aluminiumhydroxid, Kalziumsilikat und Polyamidpulver oder Gemische dieser Stoffe.

Die Tabletten, Pillen oder Granulatkörper können nach üblichen Verfahren wie Preß-, Tauch- oder Wirbelbettverfahren oder Kesseldragierung hergestellt werden und enthalten Trägermittel und andere übliche Hilfsstoffe wie Gelatine, Agarose, Stärke (z.B. Kartoffel-, Mais- oder Weizenstärke), Zellulose wie Ethylzellulose, Siliziumdioxid, verschiedene Zucker wie Milchzucker, Magnesiumcarbonat und/oder Kalziumphosphate. Die Dragierlösung besteht gewöhnlich aus Zucker und/oder Stärkesirup und enthält meistens noch Gelatine, Gummi arabicum, Polyvinylpyrrolidon, synthetische Zelluloseester, oberflächenaktive Substanzen, Weichmacher, Pigmente und ähnliche Zusätze entsprechend dem Stand der Technik. Zur Herstellung der Arzneiformen kann jedes übliche Fließregulierungs-, Schmier- oder Gleitmittel wie Magnesiumstearat und Trennmittel verwendet werden.

Bevorzugt haben die Zubereitungen die Form von Mantel-/Kern-Tabletten oder Mehrschichttabletten, wobei sich die Wirkkomponente 2 im Mantel bzw. im Kern bzw. in einer Schicht befindet, während sich die Wirkkomponente 1 im Kern bzw. im Mantel bzw. in einer anderen Schicht befindet. Die Wirkstoffkomponenten können auch in retadierter Form vorliegen oder an Retadierungsmaterial adsorbiert bzw. im Retadierungsmaterial (z.B. solches auf Zellulose- oder Polystyrolharzbasis, z.B. Hydroxyethylzellulose) eingeschlossen sein. Eine verzögerte Freisetzung der Wirkstoffe kann auch erreicht werden, indem die betreffende Schicht bzw. das Kompartiment mit üblichen magensaftunlöslichen Überzügen versehen wird.

Die anzuwendende Dosierung ist selbstverständlich abhängig von verschiedenen Faktoren wie dem zu behandelnden Lebewesen (d.h. Mensch oder Tier), Alter, Gewicht, allgemeiner Gesundheitszustand, dem Schweregrad der Symptome, der zu behandelnden Erkrankung, eventuellen Begleiterkrankungen, (falls vorhanden) der Art der begleitenden Behandlung mit anderen Arzneimitteln, oder der Häufigkeit der Behandlung. Die Dosierungen werden im allgemeinen mehrfach pro Tag und vorzugsweise einmal bis dreimal pro Tag verabreicht. Die verwendeten Mengen an Einzelwirkstoff orientieren sich hierbei an der empfohlenen Tagesdosis des jeweiligen Einzelwirkstoffs und sollen im allgemeinen im Kombinationspräparat von 10 % bis 100 % der empfohlenen Tagesdosis liegen, bevorzugt von 20 % bis 80 %, insbesondere bei 50 %. Die geeignete Therapie mit den erfindungsgemäßen Kombinationen besteht somit z.B. in der Verabreichung von einer, zwei oder 3 Einzeldosierungen der erfindungsgemäßen Kombinationspräparate bestehend aus
1) 1 mg bis 10 mg, vorzugsweise von 2 mg bis 5 mg Vinpocetin und
2) 20 mg bis 80 mg, vorzugsweise 30 mg bis 50 mg Diltiazem,
wobei die Menge naturgemäß von der Zahl der Einzeldosierungen und auch der zu behandelnden Krankheit abhängig ist und eine Einzeldosierung auch aus mehreren, gleichzeitig verabreichten Dosiereinheiten bestehen kann. Die Dosis beträgt bei einem Erwachsenen bei oraler Applikation im allgemeinen für Vinpocetin (Komponente 1) höchstens 30 mg/Tag und für Diltiazem (Komponente 2) höchstens 240 mg/Tag.

Für alle in vitro-Tests wurden periphere mononukleäre Zellen (PBMC) aus dem Blut gesunder Spender durch Dichtegradient-Zentrifugation in Ficoll-Hypaque (Pharmacia, Uppsala) gewonnen. Durch diesen Isolierungsschritt wurden Monozyten und Lymphozyten von den übrigen Blutbestandteilen abgetrennt. Die weitere Aufarbeitung dieser PBMC-Fraktion erfolgte gemäß den Anforderungen der nachfolgend beschriebenen Testansätze.

### Beispiel 1

Nach Waschen der PBMC in Hank's-Lösung (HBSS) wurde die Zellzahl mit Kulturmedium (RPMI-1640, versetzt mit 10 % (v/v) hitzeinaktiviertem fötalem Kälberserum und 1 % (v/v) Penicillin/Streptomycin (Biochrom, Berlin)) auf 1 x 10⁶ Zellen/ml eingestellt. Die so erhaltenen PBMC wurden zusammen mit Phythämagglutinin (PHA, 5 *µ*g/ml) auf Mikrotiterplatten inkubiert. Die Testsubstanzen Dibutyryl-cAMP (db-cAMP) oder Nifedipin (Sigma, München) wurden in Konzentrationen von 0,01 *µ*M bis 100 *µ*M (Endkonzentration) allein oder in Kombination in HBSS zugegeben. Die Inkubation erfolgte bei 37°C in 5 % CO₂ unter Lichtschutz. Nach 20 Stunden (h) (TNFα-Bestimmung), 24 h (IL-2-Bestimmung) oder 120 h (IFN_{γ}-Bestimmung) wurden die Zellüberstände auf Zytokin-Freisetzung hin getestet. Die TNFα-Bestimmung erfolgte über dessen Cytotoxizität gegenüber L929-Zellen und IL-2 wurde über die Proliferation von CTL-L6-Zellen quantifiziert, während für die Bestimmung von IFN_{*y*} ein Sandwich-ELISA verwendet wurde.
Tabelle 1 zeigt die Hemmung der PHA-induzierten Freisetzung von Zytokinen (TNFα, IL-2, IFN_{γ}) aus peripheren mononukleären Zellen duch exogene Zugabe von membrangängigem cAMP (Dibutyryl-cAMP), einem PDE-Hemmer (Pentoxifyllin) und einem Ca²⁺-Kanal Blocker (Nifedipin) einzeln oder in Kombination:

**Tabelle 1:**

| Testsubstanz | Endkonzentration | TNFα | IL-2 % Hemmung | IFNγ |
|---|---|---|---|---|
| Nifedipin | 10 *µ*Mol/l | 36±9 | 23±11 | 16±6 |
| Pentoxifyllin | 50 *µ*Mol/l | 28 ±10 | 14±9 | 20 ±9 |
| db-cAMP | 0,1 *µ*Mol/l | 31±13 | 12±4 | 15±8 |
| Nifedipin | 10 *µ*Mol/l | | | |
| + Pentoxifyllin | 50 *µ*Mol/l | 82±13 | 66±15 | 75±14 |
| | | | | |
| Nifedipin | 10 *µ*Mol/l | | | |
| + db-cAMP | 0,1 *µ*Mol/l | 87±17 | 81 ±14 | 74±16 |

Beispiel 1 zeigt anhand des klassischen Kalziumkanal-Blockers Nifedipin, daß eine Kombination mit dem Phosphodiesterase-Hemmer Pentoxifyllin die Bildung verschiedener für die Immunantwort relevanter Zytokine wie Tumor-Nekrose-Faktorα (TNFα), Interleukin-1 (IL-1) oder γ-Interferon (IFNγ) überadditiv hemmt, so daß auch für in vivo-Bedingungen ein überadditiver immunsuppressiver Effekt der Kombination erwartet werden kann. Setzt man anstelle von Pentoxifyllin eine membrangängige Form von cAMP (Dibutyryl-cAMP) ein, so sind ähnlich überadditive Effekte festzustellen, was darauf hinweist, daß im Falle von Pentoxifyllin unter den gewählten experimentellen Bedingungen tatsächlich der PDE-Hemmeffekt dieser Substanz (mit einer daraus resultierenden intrazellulären Erhöhung von cAMP) eine Rolle spielt mit der Konsequenz, daß diese Effekte auch auf andere PDE-Hemmer übertragbar sind.

### Beispiel 2

Im folgenden Test wurde die Abhängigkeit der Hemmung der PHA-induzierten Zytokin-Bildung von der Art des zugesetzten sogenannten "Kalzium-Antagonisten" untersucht. Der experimentelle Testansatz entsprach völlig dem von Beispiel 1. Die Testsubstanzen Pentoxifyllin, Nifedipin, Verapamil, Diltiazem (Sigma, München) wurden in Endkonzentrationen von 0,01 *µ*Mol bis 100 *µ*Mol zugesetzt, für Cyclosporin A (Sandoz, Basel) erstreckte sich der Endkonzentrationsbereich von 5 ng/ml bis 50 ng/ml. Als repräsentatives Zytokin wurde TNFα ausgewählt, dessen Quantifizierung wie in Beispiel 1 erfolgte.
Tabelle 2 zeigt die prozentuale Hemmung der PHA-induzierten Freisetzung von TNFα aus peripheren mononukleären Zellen in Gegenwart verschiedener "Kalzium-Antagonisten" einzeln oder in Kombination mit Pentoxifyllin.

**Tabelle 2:**

| | Nifedipin 10 *µ*Mol/l | Diltiazem 10 *µ*Mol/l | Verapamil A 10 *µ*Mol/l | Cyclosporin 25 ng/ml |
|---|---|---|---|---|
| Testsubstanz allein | 36±9 | 39±13 | 26±12 | 19±10 |
| Pentoxifyllin 50 *µ*Mol/l | 28 ± 10 | | | |
| Testsubstanz + Pentoxifyllin (50*µ*Mol/l) | 82±13 | 87±21 | 63±15 | 65±19 |

Beispiel 2 zeigt au-, daß auch das in Beispiel 1 eingesetzte Nifedipin ledig ich repräsentativ für andere sogenannte "Kalzium-Antagonisten" steht und durch z.B. Diltiazem, Verapamil oder auch Cyclosporin A ersetzt werden kann.

### Beispiel 3

Die PBMC-Fraktion wurde noch weiter fraktioniert, um reine T-Lymphozyten zu gewinnen. Hierfür wurde Lympho-KWIK-T, eine Mischung monoklonaler Antikörper aus anti-Monozyten und anti-B-Zellen sowie Complement, zusammen mit einer Mischung aus monoklonalen anti-NK- und anti-Monozyten-Antikörpern mit Leu 11 b (anti-CD16, IgM) und Leu 7 (anti-CD57, IgM) eingesetzt (One Lambda Inc., Los Angeles, California, USA). Die Isolierung und Reinigung der T-Lymphozyten aus der PBMC-Fraktion wurde mittels folgender Verfahrensschritte erreicht:
- Monozyten werden durch kalte Agglutination entfernt.
- 30 x 10⁶ erhaltene Zellen werden nnit 0,8 ml Lympho-KWIK-T für 1 Stunde bei 37°C inkubiert.
- Die Zellen werden dreimal gewaschen und anschließend für 30 Minuten bei 4°C mit der monoklonalen anti-NK- und anti-Monozyten-Antikörper-Mischung inkubiert.
- Die Zellen werden einmal gewaschen und erneut mit 0,8 ml Lympho-KWIK-T für 1 Stunde bei 37°C inkubiert.
- Es wird dreimal gewaschen und in Kulturmedium (RPMI 1640; Gibco, Life Techn. GmbH) resuspendiert.

Die so gewonnene Zellpräparation enthielt mehr als 97 % T-Lymphozyten (CD3+), wie durch Flowcytometrie bestimmt wurde. Testdurchführung:
5 x 10⁴ T-Lymphozyten wurden in 200 *µ* l Kulturmedium (RPMI 1640, versetzt mit 10 % fötalem Rinderserum, Penicillin und Streptomycin) in Mikrotiterplatten mit flachem Boden mit monoklonalen anti-CD3- bzw. anti-CD28-Antikörpern (jeweils 1 *µ*g/ml von jedem der monoklonalen Antikörper) und Phorbolmyristat-Acetat (PMA; Sigma, Schweiz; 5 ng/ml) in 5 % CO₂-Atmosphäre bei 37°C inkubiert. Die Testsubstanzen (Cyclosporin A 40 nMol/l bis 100 nMol/l, Pentoxifyllin 2 *µ*Mol/l bis 80 *µ*Mol/l, dbcAMP 0,01 *µ*Mol/l bis 50 *µ*Mol/l) wurden einzeln oder in Kombination zum Zeitpunkt 0 hinzugefügt. Nach 64 h Inkubation wurden die Zellen mit jeweils 1 *µ*Ci ³H-Thymidin versetzt, 8 h später auf Glasfaserfiltern gesammelt und die inkorporierte Radioaktivität in einem β-Zähler gemessen.

Die prozentuale Hemmung der T-Lymphozyten-Proliferation durch die Testsubstanzen wurde auf eine Kontrolle ohne Prüfsubstanz unter Abzug eines Basalwertes ohne monoklonalen Antikörper bezogen. Für die Auswertung wurde der Mittelwert aus 4 parallelen Ansätzen verwendet. Tabelle 3 zeigt die Hemmung der T-Lymphozyten-Proliferation durch die Testsubstanzen einzeln oder in Kombination in Abhängigkeit von der Art der Lymphozyten-Aktivierung.

**Tabelle 3:**

| | | Stimulierung über | |
|---|---|---|---|
| | | CD3/PMA | CD28/PMA |
| | | % Proliferationshemmung | |
| Cyclosporin A | 20 nMol/l | 29±4 | 9±5 |
| Pentoxifyllin | 40 *µ*Mol/l | 21 ±7 | 28 ± 8 |
| db-cAMP | 0,1 *µ*Mol/l | 16±6 | 11±6 |
| Cyclosporin A | 20 nMol/l | | |
| + Pentoxifyllin | 40 *µ*Mol/l | 74 ±14 | 68 ±12 |
| Cyclosporin A | 20 nMol/l | | |
| + db-cAMP | 0,1 *µ*Mol/l | 63±12 | 27±13 |

Beispiel 3 ist ein Beleg dafür, daß Pentoxifyllin nicht nur als PDE-Hemmer wirkt, indem es cAMP erhöht, sondern daß es auch eine cAMP-unabhängige Wirkkomponente aufweist. Werden T-Lymphozyten nämlich nicht über den sowohl Cyclosporin A- als auch cAMP-sensitiven CD3-abhängigen Mechanismus, sondern über den alternativen CD28-abhängigen Mechanismus aktiviert, so zeigt Pentoxifyllin in beiden Fällen gleichartige Effekte, obwohl cAMP unter den letztgenannten Bedingungen praktisch wirkungslos bleibt. Interessant ist in diesem Beispiel darüberhinaus der Befund, daß Cyclosporin A, dessen Unwirksamkeit als Monosubstanz bei Stimulierung des T-Lymphozyten über CD28 bekannt ist, in der Kombination mit Pentoxifyllin dennoch erneut einen überadditiven Gesamteffekt wie bei Stimulierung über CD3 aufweist, was darauf hindeutet, daß sich in der Kombination die Effekte nicht nur quantitativ, sondern auch qualitativ ausgeweitet haben.

### Beispiel 4

In einem parallelen intraindividuellen Vergleich wurde der Effekt der topischen Anwendung einer erfindungsgemäßen Kombination bei einem Patienten mit ausgeprägter Psoriasis untersucht. Hierfür wurden die Testsubstanzen Pentoxifyllin und Diltiazem, gelöst in 30 ml Polypropylenglycol/Ethanol/Wasser (1:1:1, v/v), einzeln oder gemeinsam in einer Endkonzentration von jeweils 2 % (Pentoxifyllin) bzw. 1 % (Diltiazem) in ein handelsübliches cremiges Hautpflegemittel (Nivea-Lotion) eingebracht, so daß letztendlich die folgenden 4 verschiedenen Zubereitungen vorlagen:
A) Lotion ohne Testsubstanz (= Vehikel-Kontrolle)
B) Lotion mit 2 % Pentoxifyllin
C) Lotion mit 1 % Diltiazem
D) Lotion mit 2 % Pentoxifyllin + 1 % Diltiazem

Diese 4 Zubereitungen wurden 2 x täglich 6 Wochen lang auf zuvor genau definierte, von ausgeprägter Psoriasis-Symptomatik betroffene Hautareale am Rumpf des Patienten lokal aufgebracht und eingerieben, wobei besonderer Wert darauf gelegt wurde, daß dieselbe Hautpartie stets nur mit derselben Zubereitung in Berührung kam. Das Therapieergebnis wurde optisch verfolgt und anhand des folgenden subjektiven Scores bewertet:

| | | |
|---|---|---|
| Symptomatik | völlig verschwunden | 5 |
| | nahezu verschwunden | 4 |
| | deutlich gebessert* | 3 |
| | geringfügig gebessert* | 2 |
| | kaum gebessert* | 1 |
| | gleich geblieben* | 0 |
| | geringfügig verschlechtert* | -1 |
| | deutlich verschlechtert* | -2 |

| | | |
|---|---|---|
| *Bezugspunkt war stets der ursprüngliche Ausgangszustand (d.h. Woche 0) | | |

Tabelle 4 zeigt das Behandlungsergebnis für alle 4 Zubereitungen gemäß dem obengenannten Bewertungs-Score über den Verlauf einer 6-wöchigen Therapie.

**Tabelle 4:**

| | Woche | | | | | | |
|---|---|---|---|---|---|---|---|
| Lotion | 0 | 1 | 2 | 3 | 4 | 5 | 6 |
| A | - | 0 | 1 | 1 | 1 | 1 | 1 |
| B | - | 0 | 1 | 1 | 2 | 2 | 2 |
| C | - | 0 | 0 | 1 | 0 | 1 | 1 |
| D | - | 1 | 1 | 2 | 3 | 3 | 4 |

Beispiel 4 zeigt, daß die erfindungsgemäße Kombination nicht nur in vitro, sonder auch in vivo am Menschen wirksam ist. Die topische Verabreichung von Pentoxifyllin (sogenannter "PDE-Hemmer") und Diltiazem (sogenannter "Kalzium-Antagonist") allein oder in Kombination zeigte deutliche Unterschiede zwischen Vehikel (= Placebo), den beiden Monosubstanzen und der Kombination in einem parallelen intraindividullen Vergleich bei Psoriasis. Während nach 6-wöchiger Einwirkung sowohl von Vehikel als auch von Diltiazem praktisch kein Unterschied gegenüber der Ausgangssituation festzustellen war und auch unter Pentoxifyllin allein sich die Symptome nur mäßig gebessert hatten, zeigte die mit der Kombination behandelte Hautpartie ein weitgehendes Abklingen aller mit Psoriasis verbundenen Symptomatik; lediglich eine Reströtung der betroffenen Hautareale war längere Zeit noch festzustellen, die jedoch unter fortgeführter Behandlung nach und nach ebenfalls weiter abnahm und nach 3 Monaten praktisch nicht mehr wahrnehmbar war.

## Patentansprüche

1. Verwendung von
a) mindestens einer Verbindung, die eine Phosphodiesterase-Hemmwirkung aufweist, ausgewählt aus der Gruppe Phenothiazin, Vinpocetin, Cilostamin, Milrinon, Trequinsin, Indolidan, Quazinon, Ro 201724, Rolipram, Zaprinast, Dipyridamol, Papaverin oder aus Xanthinderivaten wie Pentoxifyllin, IBMX oder Theophyllin und
b) mindestens einer Verbindung, die den biologisch effektiven intrazellulären Ca²⁺-Gehalt vermindert, ausgewählt aus der Gruppe Dihydropyridine wie Nifedipin, Nicardipin, Nimodipin, NZ-105, S11568, Amlodipin, Felodipin, Isradipin oder Diperdipin, Phenylalkylamine wie Verapamil, Benzothiazepine wie Diltiazem, ebenso Flunarizin, Fluspirilen, Pimozid, Fantofaron, Nicergolin, Cyclandelat oder auch Antibiotika auf Aminoglycosid-Basis wie Neomycin, Gentamycin oder Kanamycin; der Ca²⁺-Antagonisten wie 1,2-Bis-(2-aminoethoxyethan)-N,N,N',N'-tetraessigsäure; der Hemmer der intrazellulären Ca²⁺-Mobilisation wie Ryanodin, Dantrolen, TMB-8 oder HA 1077; der Calmodulin-Inhibitoren wie Naphthalinsulfonsäurederivate, Calmidazolium, Fluphenazin oder Tacrolimus
zur Herstellung eines Arzneimittels mit überadditiver Steigerung der immunsuppressiven Wirkung zur gleichzeitigen, getrennten oder zeitlich abgestuften Anwendung.

2. Verwendung gemäß Anspruch 1, **dadurch gekennzeichnet, daß** Pentoxifyllin und Nifedipin, Pentoxifyllin und Diltiazem, Pentoxifyllin und Verapamil oder Vinpocetin und Diltiazem eingesetzt wird.

3. Verwendung gemäß Anspruch 1 oder 2, **dadurch gekennzeichnet, daß** es zur Behandlung von akuten immunologischen Ereignissen wie Sepsis, Allergie, Graft-versus-Host-Reaktionen, Host-versus-Graft-Reaktionen, von Autoimmunerkrankungen wie rheumatoider Arthritis, systemischem Lupus erythematodes oder multipler Sklerose, von Psoriasis, Dermatitis, Asthma, Urtikaria, Rhinitis, Uveitis, Typ II-Diabetes, zystischer Fibrose, Colitis oder Leberfibrose eingesetzt wird.

## Claims

1. Use of
a) at least one compound which has a phosphodiesterase-inhibiting action, selected from the group consisting of phenothiazine, vinpocetine, cilostamide, milrinone, trequinsine, indolidane, quazinone, Ro 201724, rolipram, zaprinast, dipyridamol, papaverine or xanthine derivatives such as pentoxifylline, IBMX or theophylline, and
b) at least one compound which reduces the biologically effective intracellular Ca²⁺ content, selected from the group consisting of dihydropyridines such as nifedipine, nicardipine, nimodipine, NZ-105, S11568, amlodipine, felodipine, isradipine or diperdipine, phenylalkylamines such as verapamil, benzothiazepines such as diltiazem, flunarizine, fluspirilene, pimozide, fantofarone, nicergoline or cyclandelate, or aminoglycoside-based antibiotics such as neomycin, gentamycin or kanamycin; Ca²⁺ antagonists such as 1,2-bis(2-aminoethoxyethane)-N,N,N',N'-tetraacetic acid; inhibitors of intracellular Ca²⁺ mobilization, such as ryanodine, dantrolene, TMB-8 or HA1077; and calmodulin inhibitors such as naphthalenesulfonic acid derivatives, calmidazolium, fluphenazine or tacrolimus, for the preparation of a drug affording a superadditive increase in the immunosuppressant action, to be used simultaneously, separately or at different times.

2. Use according to claim 1, **characterized in that** pentoxifylline and nifedipine, pentoxifylline and diltiazem, pentoxifylline and verapamil or vinpocetine and diltiazem are used.

3. Use according to claim 1 or 2, **characterized in that** it is used for the treatment of acute immunological events such as sepsis, allergy, graft-versus-host reactions or host-versus-graft reactions, autoimmune diseases such as rheumatoid arthritis, systemic lupus erythematosus or multiple sclerosis, and psoriasis, dermatitis, asthma, urticaria, rhinitis, uveitis, type II diabetes, cystic fibrosis, colitis or fibrosis of the liver.

## Revendications

1. Utilisation de
a) au moins un composé, qui présente une activité inhibitrice de la phosphodiestérase, choisi parmi la phénothiazine, la vinpocétine, la cilostamine, le milrinone, le tréquinsin, l'indolidan, le quazinon, Ro 201724, le rolipram, le zaprinast, le dipyridamole, la papavérine ou de dérivés de xanthine comme la pentoxifylline, l'IBMX ou la théophylline, et
b) au moins un composé qui diminue la concentration intracellulaire biologiquement efficace de Ca²⁺, choisi parmi les dihydropyridines comme la nifédipine, la nicardipine, la nimodipine, NZ-105, S11568, l'amlodipine, la felodipine, l'isradipine ou la diperdipine, les phénylalkylamines comme le vérapamil, les benzothiazépines comme le diltiazem, également la flunarizine, le fluspirilen, le pimozide, le fantofaron, la nicergoline, le cyclandélate ou encore des antibiotiques à base d'amino-glycosides comme la néomycine, la gentamycine ou la kanamycine ; les antagonistes de Ca²⁺ comme l'acide 1,2-bis-(2-aminoéthoxyéthane)-N,N,N',N'-tétraacétique ; l'inhibiteur de la mobilisation intracellulaire de Ca²⁺ comme la ryanodine, le dantrolène, TMB-8 ou HA 1077 ; les inhibiteurs de la calmoduline comme les dérivés de l'acide naphtalènesulfonique, le calmidazolium, la fluphénazine ou le tacrolimus
pour la préparation d'un médicament avec une augmentation suradditive de l'activité immunosuppressive pour utilisation simultanée, séparée ou étagée dans le temps.

2. Utilisation selon la revendication 1,
**caractérisée en ce qu'**on utilise la pentoxifylline et la nifédipine, la pentoxifylline et le diltiazem, la pentoxifylline et le vérapamil ou la vapocétine et le diltiazem.

3. Utilisation selon la revendication 1 ou 2,
**caractérisée en ce qu'**on utilise pour le traitement de manifestations immunologiques aiguës comme la septicémie, l'allergie, les réactions greffon vis à vis de l'hôte, hôte vis à vis du greffon, les maladies auto-immunes comme l'arthrite rhumatoïde, le *Lupus erythematodes* systémique ou les scléroses multiples, le psoriasis, l'eczéma, l'asthme, l'urticaire, la rhinite, l'uvéite, les diabètes de type II, la fibrose cystique, la colite ou la fibrose du foie.
